# EUROPEAN PATENT APPLICATION

(11) **EP 4 159 138 A1**
(43) Date of publication of application: **05.04.2023**
(21) Application number: 21200178.8
(22) Date of filing: 30.09.2021
(51) Int. Cl.: A61B 6/04, A61B 6/00, A61N 5/10, G06T 7/00

(54) **PROSPECTIVE QUALITY ASSESSMENT FOR IMAGING EXAMINATION PRIOR TO ACQUISITION**

(71) Applicant: Koninklijke Philips N.V., 5656 AG Eindhoven (NL)
(72) Inventor: GOOßEN, André, Eindhoven (NL); YOUNG, Stewart Matthew, Eindhoven (NL); KRÖNKE, Sven, Eindhoven (NL); VON BERG, Jens, Eindhoven (NL); HARDER, Tim Philipp, Eindhoven (NL); BYSTROV, Daniel, Eindhoven (NL)
(74) Representative: Philips Intellectual Property & Standards

(57) **Abstract**

The present invention relates to medical imaging. In order to reduce repeat images, it is proposed to enable automated prediction of quality metrics prior to image formation by exploiting data from sensors. This may greatly improve the quality of medical image data acquired in the actual imaging examination, thereby leading to fewer retakes, less delayed treatment to patients, shortened workflow, and higher patient rate. In X-ray and CT exams, fewer retakes may also reduce radiation doses for patients.

## Description

### FIELD OF THE INVENTION

The present invention relates to medical imaging, and particularly to a computer-implemented method, apparatus, system, and computer program for prospective quality assessment for imaging examination prior to acquisition.

### BACKGROUND OF THE INVENTION

Proper patient positioning is crucial for the diagnostic quality. Relevant quality criteria and recommendations have been formulated in official standards. In clinical routines, there are many factors causing deviations from these standards, such as high workload, lack of training, missing education, missing feedback, and lack of rewards. Image violating one or more of the quality criteria may often cause extra workload for the medical staff. They may lead to misdiagnosis and thus may induce a patient health risk.

To reduce misdiagnosis, computer-aided approaches have been developed to analyze medical image data to assess quality after the image has been taken. If the quality of the acquired medical image does not meet the quality criteria and recommendations in official standards, it may be necessary to repeat the exams. However, complications resulting from repeat imaging include delayed treatment to patients, lower patient rate, and for X-ray and CT exams higher radiation doses for patients.

### SUMMARY OF THE INVENTION

There may be a need to reduce repeat images.

The object of the present invention is solved by the subject-matter of the independent claims, wherein further embodiments are incorporated in the dependent claims. It should be noted that the following described aspects of the invention apply also for the computer-implemented method, the apparatus, the system, and the computer program.

According to a first aspect of the present invention, there is provided a computer-implemented method for prospective quality assessment for imaging examination prior to acquisition, comprising the following steps:
a) receiving sensor data of a body part of a patient to be imaged by a medical imaging apparatus using a medical imaging modality;
b) generating a quality metric from the received sensor data using a data-driven model, wherein the data-driven model has been trained based on a training dataset that comprises a plurality of training examples, each training example comprises sensor data of the body part acquired in an imaging session and an associated quality metric derived from image data acquired using the medical imaging modality in the imaging session; and
c) providing the generated quality metric for prospective quality assessment for imaging examination prior to acquisition.

In other words, a computer-implemented method is proposed to enable automated prediction of quality metrics prior to image formation, by exploiting data from sensors. The proposed method involves obtaining sensor data of a body part of a patient to be imaged during positioning/preparation of an imaging examination. The sensor data may be acquired by any suitable sensor, such as an optical sensor, a thermal sensor, a depth sensor, an array of radio frequency sensors, a fibre optical range sensor, or any combination thereof. If the patient is lying on a patient support, the sensor data may also be acquired by a sensor embedded in the patient support, such as a pressure sensor embedded in the patient support, an ultrasound sensor embedded in the patient support, etc.

A quality metric may be derived from the received sensor data using a trained data-driven model. The data-driven model has been trained to predict the quality metric of to-be-acquired medical image data directly from the sensor data input prior to the actually imaging examination. In some examples, the quality metric may be derived directly from the received sensor data. In some examples, an anatomy model of the target anatomy may be fitted to the received sensor data, and the quality metric may be derived from the fitted anatomy model.

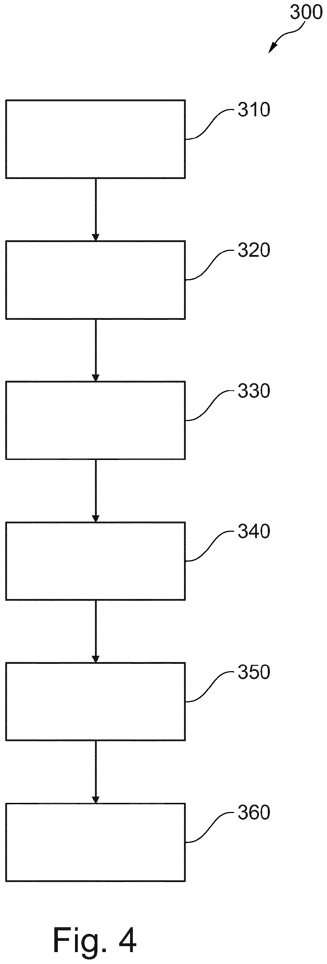

The quality metric may also be referred to as predicted quality metric. The data-driven model has been trained on a training data set that comprises a plurality training examples. Each example comprises sensor data of the body part acquired in an imaging session and an associated quality metric derived from image data acquired using the medical imaging modality in the same imaging session. The quality metric may be manually annotated by skilled clinicians and/or derived from the image data in an automated way. In general, the data-driven model has been trained to learn an association between sensor data acquired during positioning/preparation of an imaging examination and a quality metric derived from image data acquired by a medical scanner after the actual imaging examination. An exemplary training process will be explained with respect to the embodiment shown in Fig. 4.

In some examples, the quality metric may be a single value. In some examples, the quality metric may be a vector of numerical values. Each numerical value represents a deviation of a position and/or a rotation of an anatomical feature in the image data acquired using the medical imaging modality from a desired position and/or rotation of the anatomical feature.

The data-driven model may also be referred to as quality prediction model. Exemplary data-driven models may include, but are not limited to, artificial neural networks, trained random forests, and a model based segmentation approach.

The generated quality metric is then provided for prospective quality assessment for imaging examination prior to acquisition. For example, the trained data-driven model may be applied by prospectively displaying predicted quality measures during preparation/positioning of the exam. For example, the generated quality metric may be compared with a reference quality metric and an acoustic signal may be generated indicating whether the patient is correctly positioned. In a further example, the generated quality metric may be compared with a reference quality metric and the medical scanner may be triggered to perform image acquisition if the difference between the generated quality metric and the reference quality metric is less than a threshold.

The proposed method may be provided for prospective quality assessment for various imaging examinations prior to acquisition. Exemplary imaging modalities may include, but not limited to, X-ray imaging, MR imaging, CT imaging, positron-emission tomography (PET) imaging, and automatically-steered ultrasound imaging. Further examples of imaging modalities may include a combined therapy/diagnostic apparatus, such as an MR-Linac apparatus, an MR proton therapy apparatus, and/or a cone beam CT apparatus.

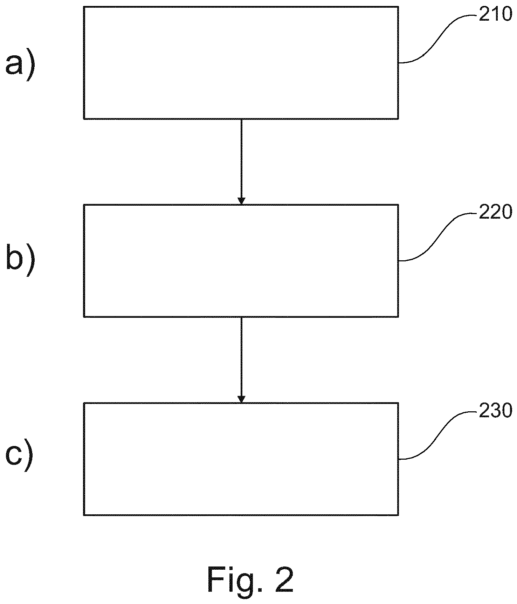

The computer-implemented method will be explained hereinafter and particularly with respect to the embodiment shown in Fig. 2.

The proposed method may allow an operator to optimize the predicted quality metric prior to taking the actual exam. For example, an instant prediction of the current positioning quality may be indicated, while moving the patient being observed from the sensor. This may greatly improve the quality of medical image data acquired in the actual imaging examination, thereby leading to fewer retakes, less delayed treatment to patients, shortened workflow, and higher patient rate. In X-ray and CT exams, fewer retakes may also reduce radiation doses for patients.

According to an embodiment of the present invention, the generation of a quality metric from the received sensor data comprises:
- generating the quality metric directly from the received the sensor data; or
- fitting an anatomy model of a target anatomy to the sensor data and generating the quality metric from the fitted anatomy model of the target anatomy.

The anatomy model of the target anatomy may be a two-dimensional model, a three-dimensional model, or a higher-dimensional model, which models joint dependent articulation (e.g. flexion) or anatomical variations.

According to an embodiment of the present invention, the computer-implemented method further comprises:
- determining whether the generated quality metric meets a predetermined criterion; and
- generating a signal indicative of whether the generated quality metric meets the predetermined criterion.

In an example, it may be determined whether a deviation between the generated quality metric and a reference quality metric is less than a threshold value.

In an example, it may be determined whether the generated quality metric is within an allowable range.

According to an embodiment of the present invention, the signal comprises a signal for controlling a device to inform an operator whether the patient is ready for image acquisition.

In an example, the device is a speaker configured to generate an acoustic signal indicating whether the patient is correctly positioned.

In another example, the device is a lighting device configured to generate a light signal indicating whether the patient is correctly positioned.

In a further example, the device is a haptic device configured to apply forces, vibrations, or motions to the patient and/or the operator indicating whether the patient is correctly positioned.

According to an embodiment of the present invention, the signal comprises a signal for triggering the medical imaging apparatus to start image acquisition.

In other words, the medical imaging apparatus may automatically start image acquisition in response to a signal indicating that the patient is correctly positioned.

According to an embodiment of the present invention, the computer-implemented method further comprises:
- receiving image data of the body part of the patient after image acquisition;
- determining, based on the received imaged data, a further quality metric; and
- determining a difference between the quality metric generated from the sensor data before image acquisition and the further quality metric derived from the image data after image acquisition; and
- further training the data-driven model using the difference.

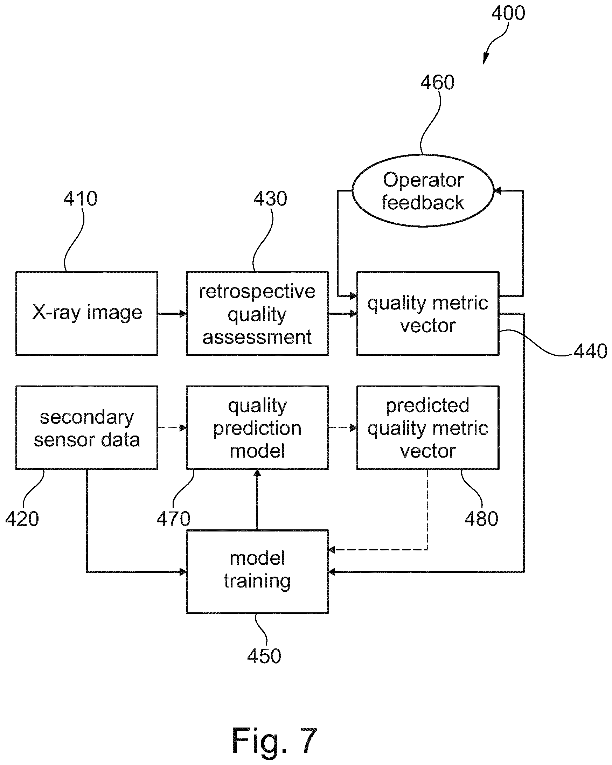

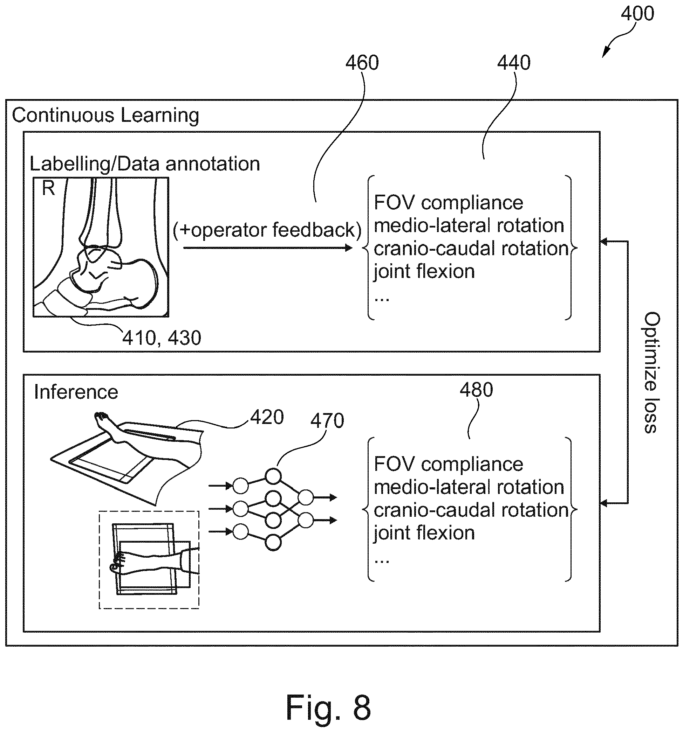

In other words, it is proposed to allow to rate the prediction by comparing it to the reference quality metrics derived later on from the final image after the actual image acquisition. This may enable a closed loop of continuous and supervised learning, enabling a strategy to continually improve the data-driven model. In addition, the closed training loop may enable the creation of models trained on very large amounts of data without compromising data privacy. This will be explained hereinafter and in particular with respect to the embodiments shown in Fig. 7 and Fig. 8.

According to an embodiment of the present invention, the computer-implemented method further comprises:
- receiving a user input indicative of a user-defined quality metric of the received image data; and
- determining a difference between the quality metric generated from the sensor data before image acquisition and the user-defined quality metric; and
- further training the data-driven model using the difference.

In other words, a user (e.g. radiographer) can review/check the quality metric vector for the exam and provide feedback, e.g. confirm/alter specific metrics. Putting this expert feedback into the continuous learning loop implements an even more reliable strongly-supervised learning strategy. This will be explained hereinafter and in particular with respect to the embodiments shown in Fig. 7 and Fig. 8.

According to an embodiment of the present invention, the quality metric is a vector of numerical values, each numerical value representing a deviation of a position and/or a rotation of an anatomical feature in the image data acquired using the medical imaging modality from a desired position and/or rotation of the anatomical feature.

Different numerical values may reflect different quality aspects. For example, as shown in Fig. 8, the X-ray image quality may be determined in terms of a quality vector with different quality aspects, such as Field-of-View (FOV) compliance, rotation, and flexion. This will be explained hereinafter and in particular with respect to the embodiment shown in Fig. 8.

According to an embodiment of the present invention, the sensor data is acquired by one or more of the following: an optical sensor, a depth sensor, a thermal sensor, a pressure sensor, an ultrasound sensor, and an array of radio frequency sensors.

According to an embodiment of the present invention, the medical imaging modality comprises one or more of: magnetic resonance imaging, ultrasound imaging, X-ray imaging, computer tomography imaging, and positron-emission tomography imaging.

According to an embodiment of the present invention, the medical imaging modality comprises a hybrid modality including one or more of: MR-Linac, MR proton therapy, and cone beam computer tomography.

According to a second aspect of the present invention, there is provided an apparatus for prospective quality assessment for imaging examination prior to acquisition, the apparatus comprising one or more processing unit(s) to generate a quality metric, wherein the processing unit(s) include instructions, which when executed on the one or more processing unit(s) perform the method steps according to the first aspect and any associated example.

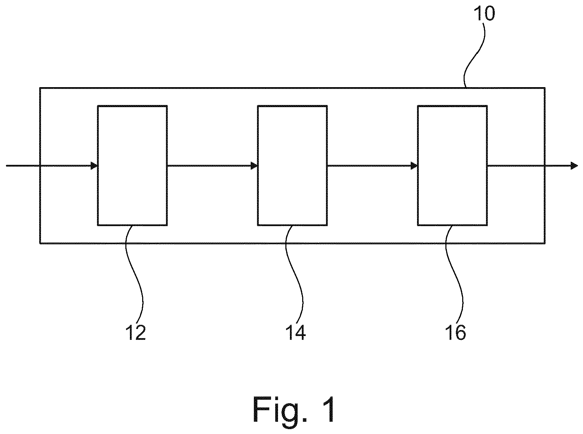

This will be explained hereinafter and particularly with respect to the embodiment shown in Fig. 1.

According to a third aspect of the present invention, there is provided a system, comprising:
- a medical imaging apparatus configured to acquire image data of a body part of a patient;
- a sensor configured to acquire sensor data of the body part of the patient; and
- an apparatus according to the second aspect and any associated example configured to receive the sensor data and provide a quality metric for prospective quality assessment for imaging examination prior to acquisition.

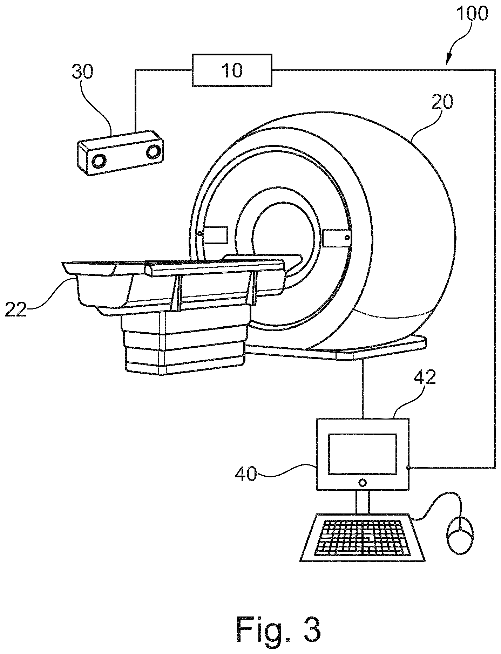

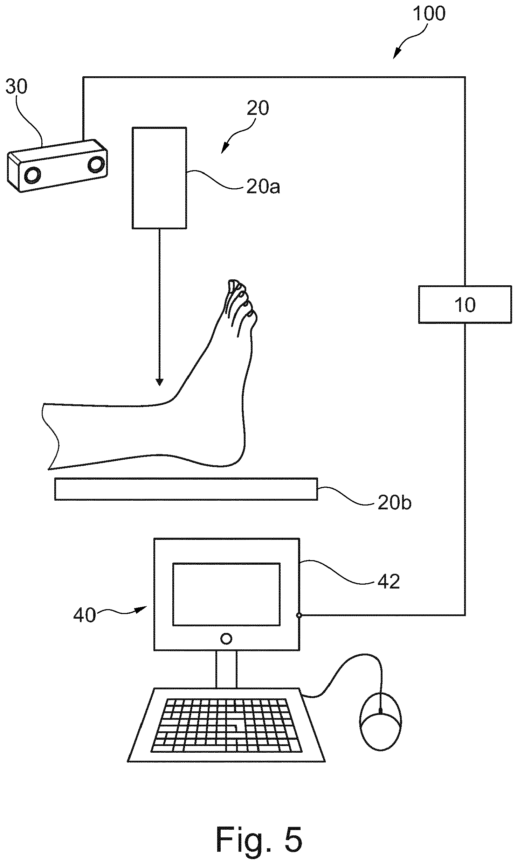

This will be explained hereinafter and particularly with respect to the embodiments shown in Fig. 3 and Fig. 5.

According to an embodiment of the present invention, the medical imaging apparatus is configured to start image acquisition according to the provided quality metric. Alternatively or additionally, the system further comprises a device configured to inform whether the patient is ready for image acquisition based on the quality metric.

In an example, the device is a speaker configured to generate an acoustic signal indicating whether the patient is correctly positioned.

In another example, the device is a lighting device configured to generate a light signal indicating whether the patient is correctly positioned.

In a further example, the device is a haptic device configured to apply forces, vibrations, or motions to the patient and/or the operator informing whether the patient is correctly positioned.

According to a further aspect of the present invention, there is provided a computer program product comprising instructions which, when the program is executed by at least one processing unit, cause the at least one processing unit to carry out the steps of the method according to the first aspect and any associated example.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 illustrates a block diagram of an exemplary apparatus.
Fig. 2 is a flow chart of an exemplary computer-implemented method.
Fig. 3 schematically illustrates an example of a system.
Fig. 4 is a flowchart of a method for generating training data for training the data-driven model.
Fig. 5 shows a further example of a system.
Fig. 6 shows an exemplary lateral image of an ankle.
Fig. 7 shows an exemplary method for implementing a weakly-supervised continuous learning.
Fig. 8 shows an example of a continuously learning framework for quality metric prediction with example data.

### DETAILED DESCRIPTION OF EMBODIMENTS

Fig. 1 illustrates a block diagram of an exemplary apparatus 10 for prospective quality assessment for imaging examination prior to acquisition. The apparatus 10 may include an input unit 12, one or more processing units 14, and an output unit 16.

In general, the apparatus 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 1 may show a limited number of components by way of example, it can be appreciated that a greater or a fewer number of components may be employed for a given implementation.

For example, the apparatus 10 may be embodied as, or in, a device or apparatus, such as a server, workstation, imaging device, or mobile device. The apparatus 10 may comprise one or more microprocessors or computer processors, which execute appropriate software. The processing unit 14 of the apparatus 10 may be embodied by one or more of these processors. The software may have been downloaded and/or stored in a corresponding memory, e.g., a volatile memory such as RAM or anon-volatile memory such as flash. The software may comprise instructions configuring the one or more processors to perform the functions described herein.

It is noted that the apparatus 10 may be implemented with or without employing a processor, and also may be implemented as a combination of dedicated hardware to perform some functions and a processor (e.g., one or more programmed microprocessors and associated circuitry) to perform other functions. For example, the functional units of the apparatus 10, e.g., input unit 12, processing unit 14, and output unit 16, may be implemented in the device or apparatus in the form of programmable logic, e.g., as a Field-Programmable Gate Array (FPGA). The input unit 12 and the output unit 16 may be implemented by respective interfaces of the apparatus. In general, each functional unit of the apparatus may be implemented in the form of a circuit.

The apparatus 10 may also be implemented in a distributed manner. For example, some or all units of the apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

The input unit 12 may include hardware and/or software to enable the apparatus 10 to receive sensor data from a sensor via a wired connection or via a wireless connection.

The processing unit(s) 14 may execute instructions to perform the method described herein, which will be explained in detail with respect to the embodiment shown in Fig. 2.

The output unit 16 may include hardware and/or software to enable the apparatus to communicate with other devices (e.g. display, storage devices, etc.) and/or a network (LTE, LAN, Wireless LAN, etc.) to provide the generated quality metric.

Fig. 2 is a flowchart of a computer-implemented method 200 for prospective quality assessment for imaging examination prior to acquisition. The method 200 is described below with reference to Fig. 3. The method 200 is not, however, limited to the example of Fig. 3.

The computer-implemented method 200 may be implemented as a device, module, or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages. For example, the computer-implemented method 200 may be implemented as the apparatus 10 shown in Fig. 1

Fig. 3 schematically shows a system 100 according to an embodiment of the present disclosure. The system 100 comprises an apparatus 10, a medical imaging apparatus 20, a patient support 22 movable along a central system axis of the medical imaging apparatus, a sensor 30, and a console 40. The medical imaging apparatus 20 is configured to acquire image data of a body part of a patient. The sensor 30 is configured to acquire sensor data of the body part of the patient. The apparatus 10 is configured to receive the sensor data and provide a quality metric for prospective quality assessment for imaging examination prior to acquisition.

Overall operation of the medical imaging apparatus 20 may be controlled by an operator from a console 40. The console 40 may be coupled to a screen or monitor 42 on which the acquired images or imager settings may be viewed or reviewed. An operator, such as a medical lab technical, can control via the console 40 an image acquisition. In the example of Fig. 3, the apparatus 10 is a separate device configured to communicate with the sensor 30 through a wireless and/or a wire-based interface. However, in alternative examples (not shown), the apparatus 10 may resident in the console 40 running as software routines.

Turning back to Fig.2, beginning at block 210, i.e. step a), an apparatus, such as apparatus 10 shown in Fig. 1, receives sensor data of a body part of a patient from a sensor that monitors the body part during positioning/preparation of an imaging examination. The body part of the patient is to be imaged by a medical imaging apparatus using a medical imaging modality. The apparatus may receive the sensor data from the sensor via a wired connection or via a wireless connection. The apparatus may have a sensor data interface to access the sensor data, which may take various forms, such as a network interface to a local area network, but also a video interface, e.g. HDMI, etc.

In the example of Fig. 3, the sensor 30 is a depth camera. The sensor 30 may also be e.g. a thermal sensor, an ultrasound sensor e.g. embedded in the patient support 22, an array of radio frequency sensors, a pressure sensor embedded in the patient support 22, an fibre optical range sensor, or any combination thereof. Although Fig. 3 only shows a single sensor, it will be appreciated that a sensor arrangement may be provided that comprises two or more sensors. In some examples, the sensor 30 may be arranged remote from the medical imaging apparatus 20, e.g. on the ceiling of the room, such that the sensor has a field of view including at least part of an area, where the patient is positioned for imaging. In some examples, the sensor, such as a pressure sensor, may be embedded in the patient support 22.

In the example of Fig. 3, the body part of the patient will be imaged by an MR imaging apparatus. Further examples of the medical imaging apparatus 20 may include, but are not limited to, an X-ray imaging apparatus, a CT imaging apparatus, a PET imaging apparatus, an automatically-steered ultrasound imaging apparatus, etc. The medical imaging apparatus 20 may also be a combined therapy/diagnostic apparatus, such as an MR-Linac apparatus, an MR proton therapy apparatus, and a cone beam CT apparatus.

Turning back to Fig. 2, at block 220, i.e. step b), the apparatus generates a quality metric from the received sensor data using a data-driven model. The data-driven model has been trained to predict a quality metric of image data to be acquired by the medical imaging apparatus from (non-ionizing) sensor data acquired by a sensor (e.g. depth camera) prior to the actual imaging examination. For example, the data-driven model may be artificial neural networks, trained random forests, a model based segmentation approach, or the like.

The apparatus may generate the quality metric directly from the received sensor data. Alternatively, the apparatus may fit an anatomy model of a target anatomy to the received sensor data, and then generate the quality metric from the fitted anatomy model of the target anatomy. The anatomy model of the target anatomy may be e.g. a two-dimensional model, a three-dimensional model, or a four-dimensional model.

The quality metric may be a vector of numerical values. Each numerical value represents a deviation of a position and/or a rotation of an anatomical feature in the image data acquired using the medical imaging modality from a desired position and/or rotation of the anatomical feature. In some examples, the numerical values may comprise ordinal values, such as 0 representing a very small deviation, 0.5 representing a small deviation, and 1 representing a large deviation. The ordinal values may be determined by comparing the deviation with one or more predetermined thresholds. In some examples, the numerical values may comprise continuous values, such as deviations in some target coordinate system, e.g. shift in [mm], rotation in degrees, FOV opening in percent, etc. Different numerical values may reflect different quality aspects. For example, as shown in Fig. 8, the X-ray image quality may be determined in terms of a quality vector with different quality aspects, such as Field-of-View (FOV) compliance, rotation, and flexion. The quality metric may also be a single value, such as a weighted sum of the above-described numerical values.

The data-driven model has been trained based on a training dataset that comprises a plurality of training examples. Each training example comprises sensor data of the body part acquired in an imaging session and an associated quality metric derived from image data acquired using the medical imaging modality in the imaging session. An exemplary training process will be discussed hereinafter and in particular with respect to the embodiment shown in Fig. 4.

Turning back to Fig. 2, at block 230, i.e. step c), the apparatus provides the generated quality metric for prospective quality assessment for imaging examination prior to acquisition.

The trained data-driven model can be inferred during positioning/preparation of an imaging examination in order to provide a predicted quality metric. This may allow an operator (e.g. radiographer) to optimize the predicted quality metric prior to taking the actual exam. For instance, an instant prediction of the current positioning quality may be indicated, while moving the patient being observed from the sensor.

In the example shown in Fig. 3, the apparatus 10 provides the generated quality metric to the console 40. On the screen 42 the generated quality metric may be viewed or reviewed. An operator can control via the console 40 an image acquisition by actuating a joystick or pedal or other suitable input means coupled to the console 40, if the generated quality metric meets a criterion.

Optionally, the apparatus 10 may determine whether the generated quality metric meets a predetermined criterion and then generate a signal indicative of whether the generated quality metric meets the predetermined criterion.

In some examples, the generated signal may comprise a signal for controlling a device to inform whether the patient is ready for image acquisition. In an example, the device may be a speaker configured to generate an acoustic signal informing the patient that the patient is correctly positioned and the body part to be imaged should be kept motionless throughout the examination. In another example, the device may be a lighting device to generate a light signal (e.g. green light) informing the patient and/or the operator that the patient is correctly positioned.

In some examples, the generated signal may comprise a signal for triggering the medical imaging apparatus to start image acquisition. In these examples, the operator does not need to manually control an image acquisition. Rather, the medical imaging apparatus 20 may be triggered to perform automated image acquisition by a signal indicating that the patient is correctly position for image acquisition. A warning signal may also be generated informing the patient to keep the body part to be imaged motionless throughout the imaging examination.

The computer-implemented method, apparatus, and system described herein may thus improve the quality of medical image data acquired in the actual imaging examination, thereby leading to fewer retakes, less delayed treatment to patients, shortened workflow, and higher patient rate. In X-ray and CT exams, fewer retakes may also reduce radiation doses for patients.

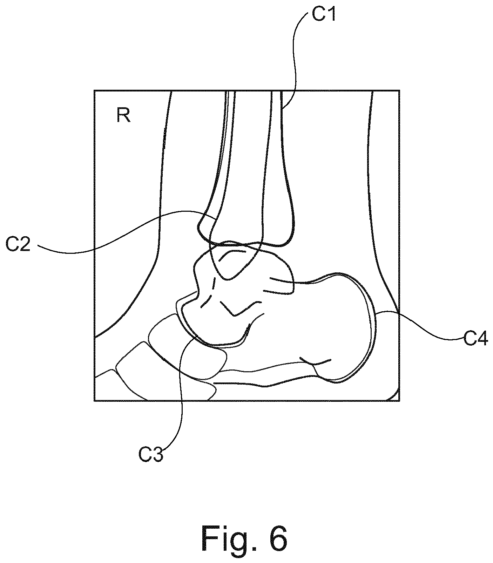

Fig. 4 is a flowchart of a method 300 for generating training data for training the data-driven model. The method 300 is described below with reference to Fig. 5 and Fig. 6. The method 300 is not, however, limited to the examples of Fig. 5 and Fig. 6.

Fig. 5 shows a further example of a system 100. In the example of Fig. 5, the system 100 comprises a medical imaging apparatus 20, which is an X-ray imaging apparatus comprising an X-ray source 20a and an X-ray detector 20b. The X-ray detector 20b is spaced from the X-ray source 20a to accommodate a body part of a patient to be imaged, such as the ankle shown in Fig. 5.

In general, during an image acquisition, a collimated X-ray beam (indicated with an arrow) emanates from the X-ray source 20a, passes through the patient at a region of interest (ROI), experiences attenuation by interaction with matter therein, and the attenuated beam then strikes the surface of the X-ray detector 20b. The density of the organic material making up the ROI determines the level of attenuation. High-density material (such as bone) causes higher attenuation than less dense materials (such as tissue). The registered digital values for the X-ray are then consolidated into an array of digital values forming an X-ray projection image for a given acquisition time and projection direction.

Overall operation of the X-ray imaging apparatus 20 may be controlled by an operator from a console 40. The console 40 may be coupled to a screen or monitor 42 on which the acquired X-ray images or imager settings may be viewed or reviewed. An operator such as a medical lab technical can control via the console 40 an image acquisition run by releasing individual X-ray exposures for example by actuating a joystick or pedal or other suitable input means coupled to the console 40.

Depending on the request, various images of the ankle can be made. A standard series may include an anteroposterior (AP) image, a Mortise image and a lateral image. When calcaneal pathology is suspected, an additional image can be made in axial direction. In the example of Fig. 5, an AP image of the ankle is acquired by the X-ray imaging apparatus 20.

The apparatus 10 may be an apparatus as described with respect to Fig. 1. In the example of Fig. 5, the apparatus 10 is a separate device configured to communicate with the sensor 30 through a wireless and/or a wire-based interface. However, in alternative examples (not shown), the apparatus 10 may resident in the console 40 running as software routines.

The sensor 30 may be a depth camera located close to or mounted to the tube head 20a. The depth camera can provide optical and depth information about the observed scene. When located close to the X-ray tube head 20a, directed towards the X-ray detector 20b and calibrated, the geometrical arrangement is known. Such sensor may be used before the acquisition to support the radiographer in assessing the patient position.

Turning back to Fig. 4, beginning at block 310, image data of a body part of a patient is received. For example, the image data of the body part may be retrieved from a database. The image data in the database may be obtained from routine examinations and/or research examinations in one or more departments or sites. The database may comprise image data of one or more body parts from one or more patients.

The image data may be previously acquired by e.g. an X-ray imaging apparatus, an MR imaging apparatus, a CT imaging apparatus, or a PET imaging apparatus. The image data may also be acquired by a combined therapy/diagnostic apparatus, such as an MR-Linac apparatus, an MR proton therapy apparatus, a cone beam CT apparatus. For example, the image data may comprise various images of the ankle, which can made by the X-ray imaging apparatus 20 shown in Fig. 5. An exemplary lateral image of the ankle is shown in Fig. 6.

At block 320, sensor data of the body part of the patient is received. The received sensor data and the received image data may be previously acquired in the same imaging examination. Sensor data may be acquired by any suitable sensor, such as an optical sensor, a depth sensor, a thermal sensor, a pressure sensor, an ultrasound sensor, an array of radio frequency sensors, or any combination thereof. For example, as shown in Fig. 5, the sensor 30 may be a depth camera for capture a depth image of the ankle.

At block 330, one or more features are extracted from the image data. The one or more features extracted from the image data may include, but are not limited to, landmarks, organ boundaries, region-of-interest, image labels in the image data acquired by the medical imaging apparatus, or any combination thereof. A fully automated procedure may be used to compute the one or more features, such as bone contours C1-C4 for tibia, fibula, talus, and calcaneus shown in Fig. 6. One option is to use an image-processing algorithm, such as an image detection algorithm that finds an approximate centre and bounding box of the one or more features in the medical image. The position and/or orientation of each extracted feature is compared with a desired position and/or orientation of the respective feature to determine a deviation of the feature. For example, the skeletal X-ray image quality may be evaluated based on field-of-view (FOV) compliance, medio-lateral rotation, cranio-caudal rotation, joint flexion, etc.

At block 340, a quality metric is generated. The quality metric may be a vector of numerical values. Each numerical value represents a deviation of a position and/or a rotation of an anatomical feature in the image data acquired using the medical imaging modality from a desired position and/or rotation of the anatomical feature. For example, the X-ray image quality may be determined in terms of a quality vector with different quality aspects, such as FOV, rotation, and flexion.

At block 350, the quality metric derived from the image data acquired by the medical imaging apparatus and the sensor data acquired by the sensor are provided as training data. Alternatively, an anatomy model of the target anatomy may be fitted to the sensor data. The quality metric derived from the image data acquired by the medical imaging apparatus and the fitted anatomy model are provided as training data

At block 360, the data-driven model is trained on the training data.

The annotated training data may be generated from routine examinations at one or more clinical sites. This continuous and unsupervised process of acquiring annotated training data provides a basis for the generation of a large ground truth database, thereby overcoming the disadvantage of the limited number of training sets obtained by a manual or semi-automated process. If data with new and not foreseen characteristics arise, or if the desired outcome of the trained system needs to be adapted, the continuously extending training set database may allow retaining of the machine-learning model for changes happening over time.

Optionally, the data-driven model may be continuously trained in the inference phase, that is, in the process of using a trained machine-learning algorithm to make a prediction. Fig. 7 shows an exemplary method 400 for implementing a weakly-supervised continuous learning.

In this example, a quality metric is generated from sensor data acquired prior to an image acquisition following the procedures indicated with dashed arrows. The quality metric may also be referred to as predicted quality metric. In particular, at block 420, the acquired sensor data is received in a way similar to the embodiment described with respect to block 210 in Fig. 2. At block 470, a quality metric is generated from the received sensor data using the trained data-driven model in a way similar to the embodiment described with respect to block 220 in Fig. 2. At block 480, the generated quality metric (i.e. the predicted quality metric) is provided for training the model.

A further quality metric is generated from image data acquired by a medical imaging apparatus after the actual imaging examination following the procedures indicated with solid arrows. The further quality metric may also be referred to as actual quality metric. In particular, at block 410, the acquired image data is received in a way similar to the embodiment described with respect to block 310 in Fig. 4. At block 430, one or more features are extracted from the image data. The position and/or orientation of each extracted feature is compared with a desired position and/or orientation of the respective feature to determine a deviation of the feature, in a way similar to the embodiment described with respect to block 330 in Fig. 4. At block 440, a quality metric is generated, which may be a vector of numerical values. Each numerical value represents a deviation of a position and/or a rotation of an anatomical feature in the image data acquired using the medical imaging modality from a desired position and/or rotation of the anatomical feature. This may be done in a way similar to the embodiment described with respect to block 340 in Fig. 4.

Optionally, at block 460, an operator (e.g. radiographer) may review and check the quality metric vector for the exam, provide feedback, e.g. confirm/alter specific metrics, and put this expert feedback into the continuous learning loop, which implements an even more reliable strongly-supervised learning strategy. For example, the apparatus 10 may receive a user input indicative of a user-defined quality metric of the received image data, determine a difference between the quality metric generated from the sensor data before image acquisition and the user-defined quality metric, and further train the data-driven model using the difference.

The difference between the predicted quality metric derived from the sensor data prior to the image acquisition and the actual quality metric derived from the image data acquired after the actual image acquisition may be used as loss function to implement a weakly-supervised continuous learning.

By configuring e.g. thresholds, the continuous learning loop can be adapted to department-specific quality requirements (e.g. the width of the field-of-view) without the need of manual annotations.

Fig. 8 shows an example of a continuously learning framework for quality metric prediction with example data. In this example, an actual quality metric is derived from image data acquired by a medical imaging apparatus (e.g. X-ray imaging apparatus shown in Fig. 5) following the procedures described with respect to blocks 410, 430, 440, and 460 in Fig. 7. A predicted quality metric is derived from sensor data acquired prior to image acquisition following the procedures described with respect to blocks 420, 470 and 480 in Fig. 7. In this example, the X-ray image quality is predicted in terms of a quality vector with different quality aspects (such as, field-of-view, rotation, and flexion). An RGB/depth camera may provide sensor data to predict quality metrics using a continuously updated data-driven model.

It should also be understood that, unless clearly indicated to the contrary, in any methods claimed herein that include more than one step or act, the order of the steps or acts of the method is not necessarily limited to the order in which the steps or acts of the method are recited.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the system type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A computer-implemented method (200) for prospective quality assessment for imaging examination prior to acquisition, comprising the following steps:
a) receiving (210) sensor data of a body part of a patient to be imaged by a medical imaging apparatus using a medical imaging modality;
b) generating (220) a quality metric from the received sensor data using a data-driven model, wherein the data-driven model has been trained based on a training dataset that comprises a plurality of training examples, each training example comprises sensor data of the body part acquired in an imaging session and an associated quality metric derived from image data acquired using the medical imaging modality in the imaging session; and
c) providing (230) the generated quality metric for prospective quality assessment for imaging examination prior to acquisition.

2. Computer-implemented method according to claim 1,
wherein the generation of a quality metric from the received sensor data comprises:
- generating the quality metric directly from the received the sensor data; or
- fitting an anatomy model of a target anatomy to the sensor data and generating the quality metric from the fitted anatomy model of the target anatomy.

3. Computer-implemented method according to claim 1 or 2, further comprising:
- determining whether the generated quality metric meets a predetermined criterion; and
- generating a signal indicative of whether the generated quality metric meets the predetermined criterion.

4. Computer-implemented method according to claim 3,
wherein the signal comprises a signal for controlling a device to inform whether the patient is ready for image acquisition.

5. Computer-implemented method according to claim 3 or 4,
wherein the signal comprises a signal for triggering the medical imaging apparatus to start image acquisition.

6. Computer-implemented method according to any one of the preceding claims, further comprising:
- receiving image data of the body part of the patient after image acquisition;
- determining, based on the received imaged data, a further quality metric; and
- determining a difference between the quality metric generated from the sensor data before image acquisition and the further quality metric derived from the image data after image acquisition; and
- further training the data-driven model using the difference.

7. Computer-implemented method according to claim 6, further comprises:
- receiving a user input indicative of a user-defined quality metric of the received image data; and
- determining a difference between the quality metric generated from the sensor data before image acquisition and the user-defined quality metric; and
- further training the data-driven model using the difference.

8. Computer-implemented method according to any one of the preceding claims,
wherein the quality metric is a vector of numerical values, each numerical value representing a deviation of a position and/or a rotation of an anatomical feature in the image data acquired using the medical imaging modality from a desired position and/or rotation of the anatomical feature.

9. Computer-implemented method according to any one of the preceding claims,
wherein the sensor data is acquired by one or more of the following: an optical sensor, a depth sensor, a thermal sensor, a pressure sensor, an ultrasound sensor, and an array of radio frequency sensors.

10. Computer-implemented method according to any one of the preceding claims,
wherein the medical imaging modality comprises one or more of:
- magnetic resonance imaging;
- ultrasound imaging;
- X-ray imaging;
- computer tomography imaging; and
- positron-emission tomography imaging.

11. Computer-implemented method according to any one of the preceding claims,
wherein the medical imaging modality comprises a hybrid modality including one or more of:
- MR-Linac;
- MR proton therapy; and
- cone beam computer tomography.

12. An apparatus (10) for prospective quality assessment for imaging examination prior to acquisition, the apparatus comprising one or more processing unit(s) to generate a quality metric, wherein the processing unit(s) include instructions, which when executed on the one or more processing unit(s) perform the method steps of any one of the preceding claims.

13. A system (100), comprising:
- a medical imaging apparatus (20) configured to acquire image data of a body part of a patient;
- a sensor (30) configured to acquire sensor data of the body part of the patient; and
- an apparatus (10) according to claim 12 configured to receive the sensor data and provide a quality metric for prospective quality assessment for imaging examination prior to acquisition.

14. System according to claim 13,
wherein the medical imaging apparatus is configured to start image acquisition according to the provided quality metric; and/or
wherein the system further comprises a device configured to inform whether the patient is ready for image acquisition based on the quality metric.

15. A computer program product comprising instructions which, when the program is executed by at least one processing unit, cause the at least one processing unit to carry out the steps of the method according to any one of claims 1 to 11.
